# EUROPEAN PATENT APPLICATION

(11) **EP 0 685 493 A1**
(43) Date of publication of application: **06.12.1995**
(21) Application number: 94108445.1
(22) Date of filing: 01.06.1994
(51) Int. Cl.: C07K 19/00, A61K 38/17, C12N 15/62

(54) **Tumor suppressor fusion proteins**

(71) Applicant: CANJI, Inc., San Diego CA 92121 (US)
(72) Inventor: Shih, Ah Shing, San Diego, CA 92129 (US); Davidson, Ross E., San Diego, CA 92126 (US); Johnson, Duane E., Encinitas, CA 92024 (US); Shepard, Michael H., Rancho Sante Fe, CA 92067 (US)
(74) Representative: VOSSIUS & PARTNER

(57) **Abstract**

The present invention provides a soluble molecular complex for introducing a cancer suppressor transcription factor to a mammalian cell, the complex comprising a fusion of a ligand for a mammalian cell membrane receptor releasibly bound to the cancer suppressor transcription factor. Further provided by the present invention are methods of treating pathologic conditions caused by gene dysfunction or alteration of gene products.

## Description

Throughout this application various publications are referenced within parentheses. The disclosures of these publications in their entireties are hereby incorporated by reference in this application in order to more fully describe the state of the art to which this invention pertains.

The retinoblastoma susceptibility (RB) gene has been cloned and extensively studied (Brugge, J. et al., "The Retinoblastoma Gene: A Prototypic Model for Tumor Suppression", Origins of Human Cancer: A Comprehensive Review; vol 1, 1 ed., pp 413-422 (Cold Spring Harbor) (1991)). The loss of functional Rb protein appears to be associated with the development of many human cancers. Among these are retinoblastoma, bladder carcinoma, breast cancer, chronic myelogenous leukemia, acute myelogenous leukemia, testicular tumors, dysplastic and cancerous ulcerative colitis, sporadic sarcomas, prostate carcinoma, osteosarcoma, small cell lung cancer and synovial sarcoma. The diverse origin of these neoplasms indicates an important and broad role for Rb in hyperproliferative diseases and particularly, in human cancer.

The mechanism by which Rb regulates the cell cycle may explain its important role in tumor formation. The RB protein has been found to associate with many cellular proteins, such as E2F, c-myc, N-myc and the cyclins (D class). E2F is a cellular transcription factor which binds many upstream promoters of cellular genes that encode products with putative functions which promote cell cycle progression. The formation of the Rb-E2F complex may affect the transcription of cell cycle related genes and thereby negatively regulate cell cycle progression.

The transforming proteins of many DNA tumor viruses ("oncoproteins") bind with and sequester the cell's pool of Rb as a means of deregulating cell growth. These proteins include adenovirus E1A, simian virus 40 large T antigen, and human papilloma virus E7 protein. These transforming proteins share the capacity to disrupt the interaction between transcription factor E2F and the dephosphorylated or hypophosphorylated form of RB and to stimulate cell proliferation. Thus, Rb may have an important role, with human papilloma viruses, in the genesis of genital warts and progression to cervical metaplasia.

It has been reported that replacement of a functional Rb gene in the Rb-negative cell lines WERI-Rb27 (retinoblastoma), Saos-2 (osteosarcoma), DU145 (prostate carcinoma), a HTB9 (bladder carcinoma) resulted in complete or partial suppression of transformed characteristics, including morphology, growth rate, soft agar colony formation, and tumor formation in nude mice. Truncated p56RB (start from amino acid #388) has a C-terminal domain which contains the oncoprotein-binding pockets (domain A,393-572; domain B, 646-772) has been shown to retain the growth-inhibitory activity of the full length RB after being micro-injected into Saos2 cells (Goodrich, D.W. et al., supra; Huang, H.-J.S. et al., "Suppression of the Neoplastic Phenotype by Replacement of the RB Gene in Human Cancer Cells", Science, 242:1563-66 (1988)).

However, single cell microinjection of tumor cells is not a practical mode for therapy of cancer. Each tumor has many cells, and tumor metastasis is common. Therefore, a method which allows systemic therapy is essential. In view of the foregoing, it would be highly desirable to have technology to deliver the p56Rb more efficiently and precisely to the target inside the cell.

The present invention provides a soluble molecular complex for introducing a cancer suppressor transcription factor to a mammalian cell, the complex comprising a fusion of a ligand for a mammalian cell membrane receptor releasibly bound to the cancer suppressor transcription factor. Further provided by the present invention are methods of treating pathologic conditions caused by gene dysfunction or alteration of gene products.
Figure 1 details the construction of fusion proteins PEA56RBD0 and PEA56RBD1.
Figure 2 details the construction of fusion protein PEA56RBD2S.
Figure 3 summarizes the final structures of the different Pseudomonas Exotoxin p56^{RB} fusion proteins.

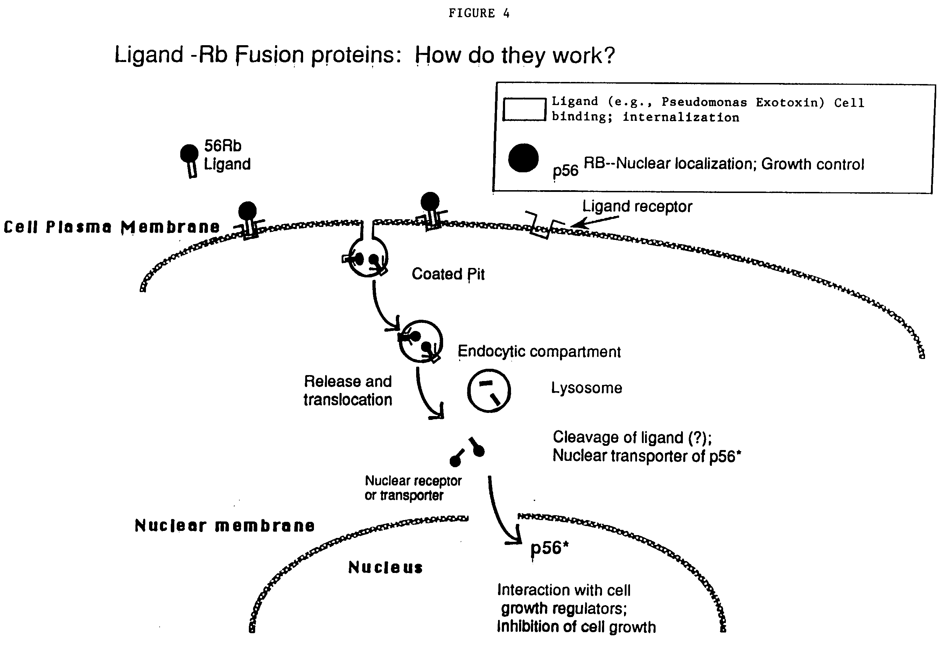

Figure 4 shows the mechanism of fusion protein interaction with the cell.
Figure 5 details the construction of fusion protein FID56RB.
Figure 6 summarizes the construction of an interferon-RB fusion protein.
Figure 7 shows the results of a Western blot analysis of the fusion proteins utilizing ∞-Rb monoclonal antibody 318 indicating that they each have the correct molecular weight and react specifically with the Rb moiety at their C-termini.
Figure 8 (Panels A and B) show SDS-PAGE gel analysis of the purified D0 and D1 proteins. Western blot analysis using ∞Rb Mab 3C8 support the identity of these proteins (Figure 8, Panels C and D).
Figure 9 shows the ability of the purified fusion proteins to bind purified viral oncoprotein SV40 T-antigen. The Tag-bound RB protein was detected by the standard chromogenic reaction using biotinylated Mab 3C8, streptavidin-POD (1:2500 dilution, Boehringer Mannheim GmbH), and ABTS (Sigma) substrate. Color development was measured at 405 nm absorbance by a Coulter microplate autoreader (Molecular Devices).
Figures 10 and 11 show the inhibition of DNA synthesis by p110^{RB} in p110^{RB}-deficient tumor cell lines .

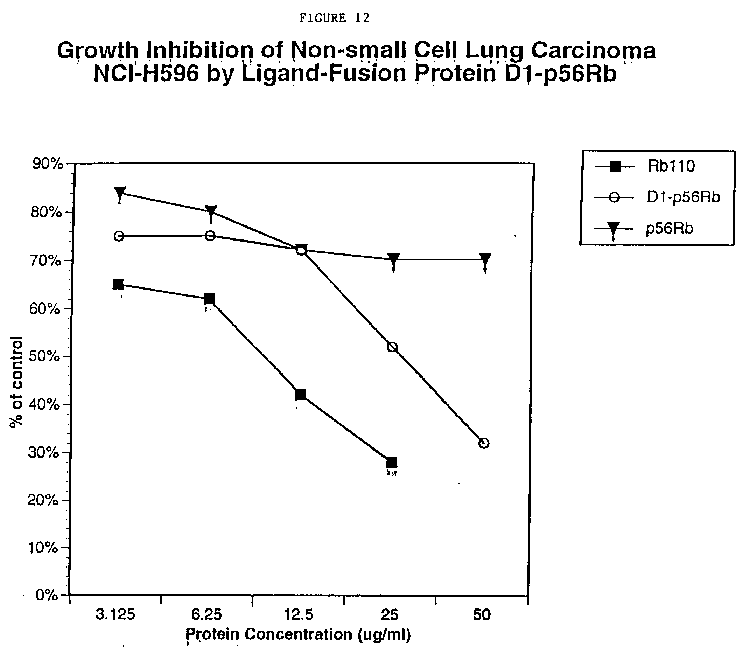

Figure 12 shows that the fusion protein D₁ PE-RB can inhibit the growth of the H596 (p110^{RB}-deficient) non-small cell lung carcinoma cell line in vitro.

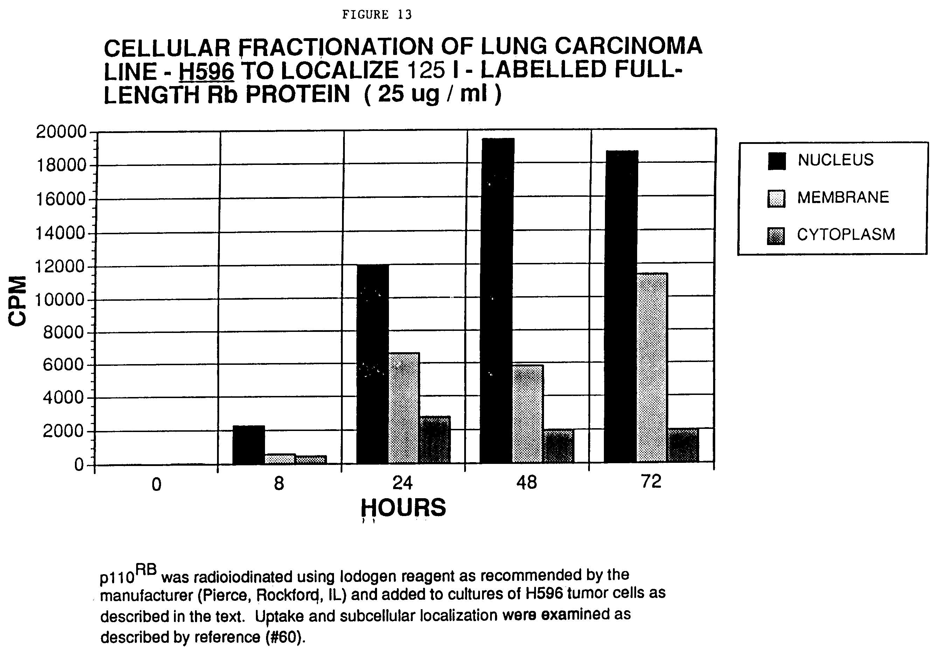

Figure 13 demonstrates that p110^{RB}-I¹²⁵ localizes to the nucleus of H596 cells in a time dependent manner. (Radke, K. et al., "Membrane Association of 36,000 Dalton substrate for tyrosine phosphorylation in Chicken Embryo Fibroblasts transformed by avian sarcoma virus", Cell Biol., 97:1601-1611 (1983)).

The present invention provides a soluble molecular complex for introducing a tumor or cancer suppressor transcription factor to a mammalian cell. As used herein, the term "tumor or cancer suppressor transcription factor" refers to proteins that act to suppress cancer or tumor growth such as Rb or p53. It is intended that the term refers to both the full native proteins or to fragments modifications or derivatives which retain the tumor suppressor activity of the native protein. It is also intended that the term encompass proteins having a nuclear localization signal, native or synthetic. For example, the nuclear localization signal present on p56 RB is sufficient to direct nuclear translocation.

This invention provides ligands to a cell membrane receptor, that will induce endocytosis and carry-in the tumor suppressor transcription factor. The ligand must be releasibly bound to the tumor suppressor transcription factor, allowing escape of the tumor suppressor transcription factor into the cytoplasm of the cell. As used herein the term "releasibly bound" is intended to encompass an extracellularly stable bond that is cleavable under appropriate conditions within the cell so as to release the tumor suppressor transcription factor intracellularly. For example, cleavage of the bond can be pH or enzymologically regulated. As used herein "ligand to a cell membrane receptor" refers to a molecule which binds to receptors located on the cell surface. Such receptors can be tissue specific or can be ubiquitously found on many or all cell surfaces. The cell membrane receptors all provide a -COOH binding site for the ligand. Examples of such receptors are GIP colon cell receptor, bombasin growth factor receptor on lung cells and ferretin.

Two ligands have been examined: Pseudomonas exotoxin A (PE) and interferon alpha (Wick, M.J. et al., "Analysis of the Structure-function relationship of Pseudomonas aeruginosa exotoxin A", Mol. Micro., 4:527-535 (1990); Rubinstein, M. et al., "The interferon receptors", Crit. Rev. Biochem., 21:249-275 (1986); Wileman, T. et al., "Receptor-mediated endocytosis", Biochem., 232(1):1-14 (1985); Yonehara, S. et al., "Cell surface receptor-mediated internalization of interferon: its relation to the antiviral activity of interferon", J. Gen. Virol., 64:2409-2418 (1983); Arnheiter, H. et al., "Microinjection of anti-interferon antibodies into cells does not inhibit the induction of an antiviral state by interferon", J.Virol., 52(1):284-07 (1984)). Domain 1 of exotoxin A is important to cell surface recognition and receptor binding while domain 2 is required for translocation out of the endosome (Liu, P.V. "The roles of various fractions of Pseudomonas aeruginosa in its pathogenesis. 3. Identity of the lethal toxins produced in vitro and in vivo", Infect. Dis., 116(4):481-9 (1966)). The fusion proteins have been constructed containing either partial domain 1, intact domain 1 or both domain 1 and 2. Both ligands, PE and interferon, were constructed as fusions with N-terminal truncated p56Rb and produced E. coli. It is significant that N-terminal truncated p56^{RB} has no activity on its own, unlike p110^{RB}, nor is p56^{RB} active when co-incubated with tumor cells. The fusion proteins so constructed were purified and tested. Unlike p56^{RB} unmodified, the p56^{RB} fusion proteins have the T-antigen binding activity and tumor growth suppression function of the full length p110^{RB}. In this invention, it is also shown that full length (unmodified) RB protein has growth suppression activity under pharmacologically acceptable conditions.

Exotoxin A of Pseudomonas aeruginosa is one member of a family of secreted bacterial toxins that are capable of covalently modifying specific target proteins within mammalian cells. Analysis of the identified domains show that the amino-terminal domain (domain 1) is involved in recognition of eukaryotic target cells. The central domain (domain 2) is involved in secretion of exotoxin A into the periplasm of Escherichia coli. Domain 2 also functions in translocation of exotoxin A from the eukaryotic endosome which containing the toxin after being internalized into susceptible eukaryotic cells via receptor-mediated endocytosis. The carboxy-terminal portion of exotoxin A (domain 3) encodes the enzymatic (toxin) activity of the molecule.

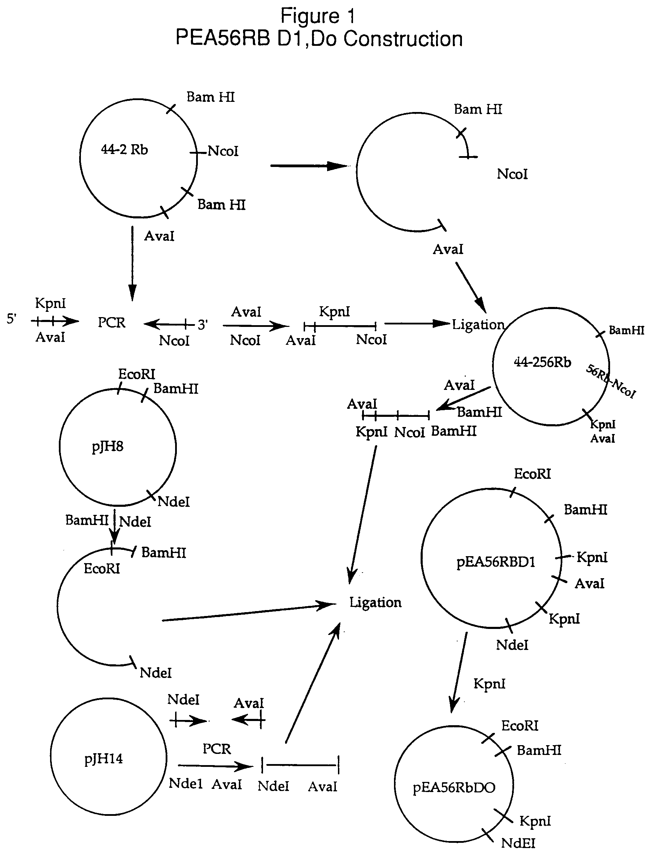

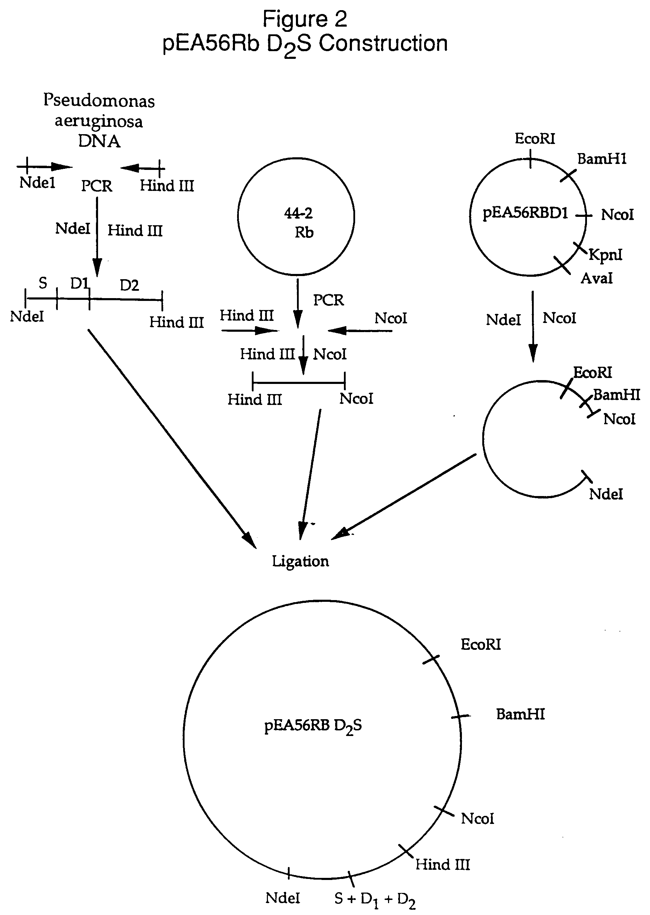

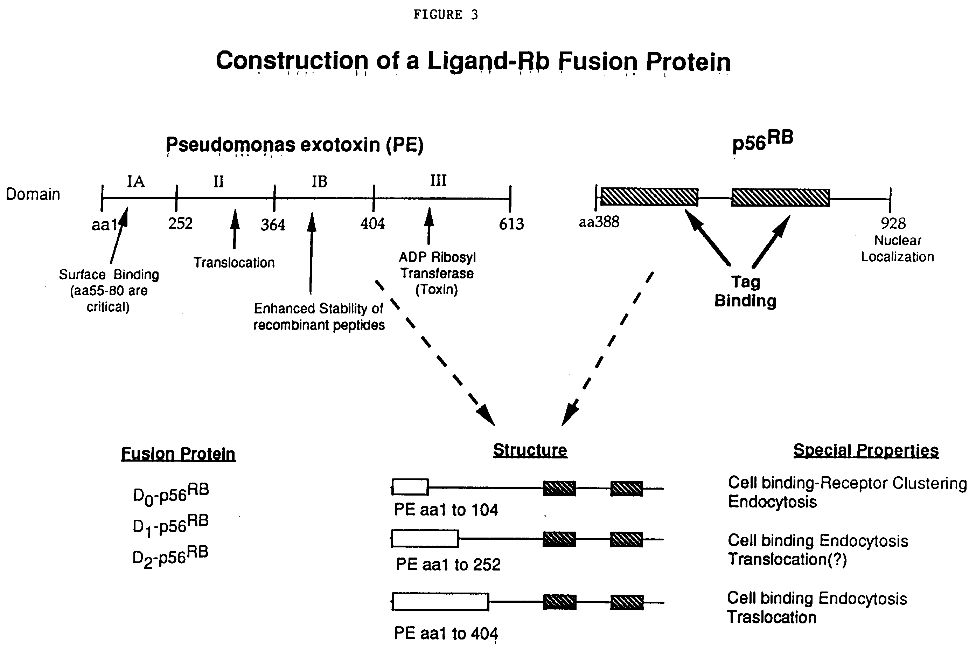

Exotoxin A enters eukaryotic cells via receptor-mediated endocytosis, is internalized into clathrin-coated pits, and proceeds into endosomes (Saelinger, C.B. et al., "Intracellular trafficking of Pseudomonas exotoxin A." Antibiot. Chemo., 39:149-59 (1987)). The receptor of exotoxin A is species-specific, and certain glycosphingolipids - asialo-GM1 and asialo-GM2, but not GM2, GM2, GM3, may act as receptors on the cell surface (Krivan, H.C. et al., "Many pulmonary pathogenic bacteria bind specifically to the carbohydrate sequence GalNAc beta 1-4Gal found in some glycolipids" Proc. Natl. Acad. Sci. USA, 85(16):6157-61 (1988)). Using the characteristics of exotoxin A, three different fusion proteins were constructed see Example I and Figure 1 (PEA56RBD0 and PEA56RBD1), Figure 2 (PEA56RBD2S). Both PJH8, PJH14 and Pseudomonas aeruginosa strains are from ATCC (Rockville, MD). Plasmid 44-2 and E. coli BL21 (with pLysS) were provided by Dr. Wen Hwa Lee. Plasmid pflag (IBI, CT) and DH5 alpha competent cells (BRL, Bethesda, MD) were obtained from suppliers. Figure 3 summarizes the final structures of the different PE p56^{RB} fusion proteins.

Another ligand tested in this invention is interferon alpha. The uptake of interferon alpha into the cell is also via receptor-mediated endocytosis. The biological activities of interferon alpha have been extensively studied and include inhibition of cell growth, induction of antiviral state and modulation of immune response.

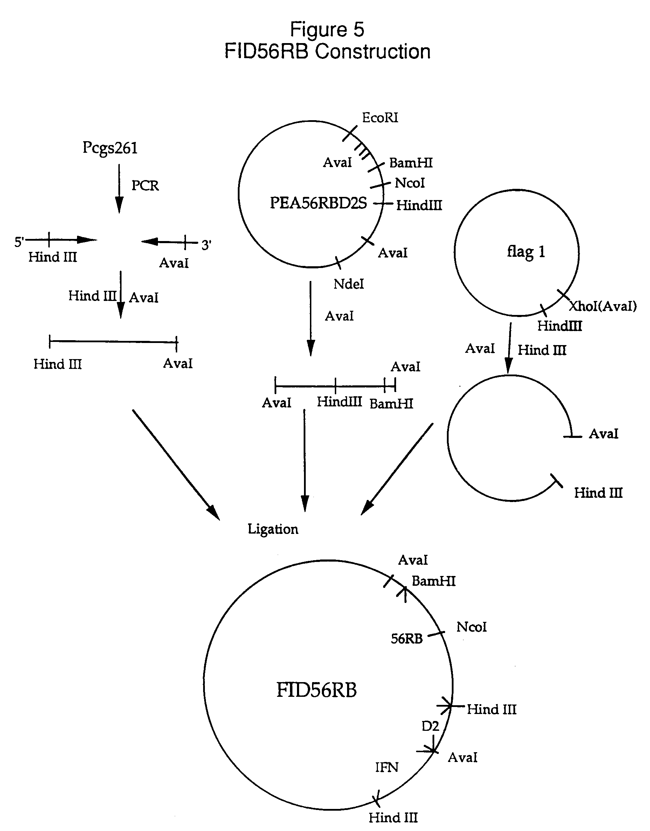

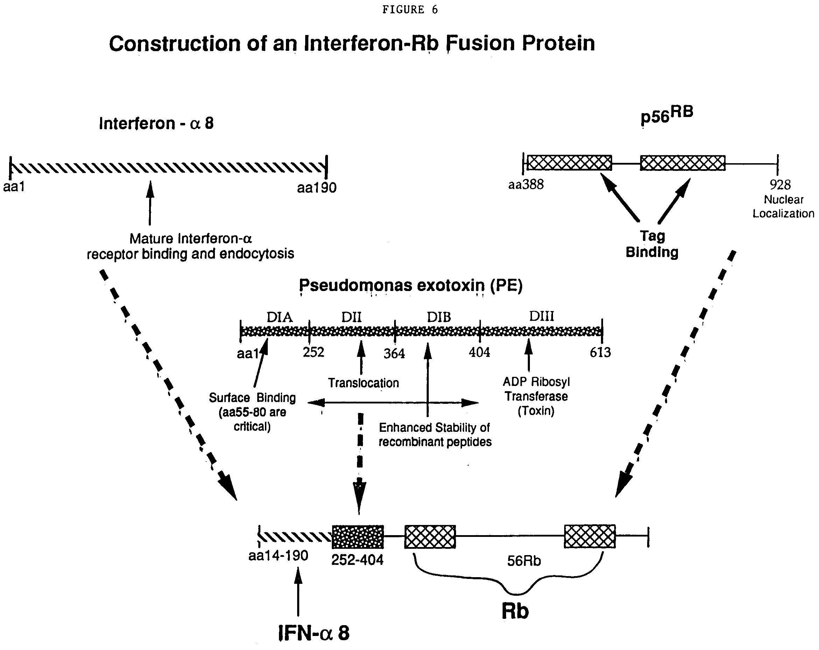

Interferon alpha has been clinically used for the treatment of hairy cell leukemia and Condylomata acuminata (venereal or genital warts). Genital warts and cervical carcinomas have been associated with human papilloma viruses. Two viral genes, E6 and E7, encode oncoproteins that, together, are sufficient for efficient immortalization of primary human squamous epithelial cells (Banks, L. et al., "Ability of the HPV16 E7 protein to bind RB and induce DNA synthesis is not sufficient for efficient transforming activity in NIH3T3 cells", Oncogene, 5:1383-1389 (1990); Heck, D.V. et al., "Efficiency of binding the retinoblastoma protein correlates with the transforming capacity of the E7 oncoproteins of the human papilloma viruses", Proc. Natl. Acad. Sci. USA, 89:4442-4446 (1992); Stirdivant, S.M. et al., "Human papilloma virus type 16 E7 protein inhibits DNA binding by the retinoblastoma gene product", Mol. Cell. Biol., 12:1905-1914 (1992); Huang, S. et al., "A cellular protein that competes with SV40 T antigen for binding to the retinoblastoma gene product", Nature, 350:160 (1991)). E7 protein binds RB protein in the C-terminal region and is required for virus replication. The interferon-RB fusion protein constructed in this invention provides synergistic anti-viral and tumor growth suppression effects and, therefore, higher therapeutic efficacy. The construction of interferon alpha-exotoxin A-domain 2-p56RB fusion protein (FID56RB) is detailed in Example I and shown in Figures 5 and 6.

The efficacy of therapeutically active proteins and peptides is often limited due to sensitivity to proteolysis, solubility, and immunogenicity. An example of a method for stabilizing such proteins is the covalent modification of the protein with a hydrophilic polymer such as polyethylene glycol (PEG) or dextran. It has been shown that such conjugation enhances solubility, decreases sensitivity to proteolysis and immunogenicity and extends the life of the protein in vivo circulation. (Beckman, J.S. et al., J. Biol. Chem., 263(14):6884 (1988)). The fusion proteins of the subject invention are intended for use with or without such modification.

It is understood that modifications which do not substantially affect the activity of the various molecules of this invention are also included within the definition of said molecules.

The following examples are intended to illustrate but not limit the present invention.

### EXAMPLE I

### Construction Of Tumor Suppressor Fusion Proteins

AvaI and NcoI Digested 44-2Rb was separated on the 1% agarose gel and the large fragment was purified with Geneclean (Bio101, San Diego, CA) method. PCR fragment from 44-2Rb using primers (1) and (3) was generated, purified after digested with AvaI and NcoI and ligated to the above large fragment to form 44-2 56Rb. AvaI-BamHI 56Rb fragment and PCR NdeI-AvaI fragment from PJH14 using primers (5) and (7) were ligated to the purified PJH8 BamHI-NdeI vector to generate PEA56RBD1. After cutting off the KpnI-KpnI fragment PEA56RBD0 was made by self-ligation.

Pseudomonas aeruginosa DNA (PE) was made by digesting the bacteria with proteinase K at 55 °C overnight, phenol/chloroform extraction and alcohol precipitation. NdeI-HindIII fragment (SD1D2) was prepared by PCR of PE with primers (4) and (6). HindIII-NcoI 56Rb 5' fragment was PCR of 44-2Rb with primers (2) and (3). These two fragments were ligated to the vector containing 3' 56Rb, generated by purifying the Ndel, NcoI digested PEA56RBD1 large fragment, to form PEA56RBD2S.

HindIII-AvaI Interferon fragment was purified from PCR of Pcgs261 with primers (8) and (9). AvaI-AvaI (XhoI) was purified from AvaI digested PEA56RBD2S by running on 1% agarose gel and Genecleaned. Flag 1 vector was purified from AvaI and HindIII digested flat 1 (IBI). After three-piece ligation, FID56RB was formed.

Primers used in the construction of the fusion proteins are the following:
(1). 5' primer for 56RB with Ava I and Kpn I site 5' AATTCCCGAGCTGGTACCGATTTTAAATTCAGCAAG 3'
(2). 5' primer for 56RB with Hind III site 5' AATTAAGCTTTCATTTTAAATTCAGCAAG 3'
(3). 3' primer for 56RB with Nco I site 5' AATTTCTAGAAAGCACATTCAGAATCCATG 3'
(4). 5' Primer for Pseudomonas aeruginosa DNA with Nde I site 5' AATTCATATGCACCTGATACCCCATTGG 3'
(5). 5' primer for PJH14 with Nde I site 5' ATTCATATGGCCGAAGAAGC 3'
(6). 3' primer for Pseudomonas aeruginosa DNA with Hind III site 5' AAGAAAGCTTTGCCGTCGCCGAGGAACTC
(7). 3' primer for PJH14 with Ava I site 5' AATTCTCGGGAAAGTGCAGGCGATGAC 3'
(8). 5' primer for pCGS261 with Hind III site 5' AGCTCTAAGCTTTGTGATCTGCCTCAGACTC 3'
(9). 3' primer for pCGS261 with Ava I site 5' AATTCTCGGGTTCCTTACTCTTCAATCT 3'
PCR reactions were performed under the following conditions:

| | |
|---|---|
| dNTP 10mM | 8 µl; |
| buffer 10x | 100 ng |
| 5' primer | 300 nM |
| 3' primer | 300 nM |
| formamide | 1.5% volume |
| Taq Polymerase | 1 unit |
| dH20 bring to | 50 µl |
| mineral oil | 50 µl |

Reaction conditions were as follows:
95° C x 5'
94° C x 1'-----55° C x 1'-----72° C x 1-2' for 30 cycles
72° C x 10'

### EXAMPLE II

### Expression and Purification of Fusion Proteins PEA56RBD0, D1, D2S, FID56RB

Based upon the BL21(DE3)pLysS host organism of Rosenberg (Rosenberg, A.H. et al., "Vectors for selective expression of clone DNAs by T7 RNA polymerase", Gene, 56(1):125-35 (1987)), after transformation with the PEA56RBD0, D1, D2S plasmid, protein production could be induced by isopropyl-β-D-thiogalactopyranoside. For FID56RB, DH5 alpha was used as the host. Bacterial overnight cultures were prepared by direct inoculation of bacteria from master seed banks into LB media containing 100 µg/mL ampicillin and 20 µg/mL chloramphenicol. After growing 15 hours at 30°C in a New Brunswick Scientific shaker, an OD₆₀₀ of between 2 to 3 was attained, the whole content of the seed flask was used to inoculate 3.4 L medium in a 5 L fermentor. The temperature of the fermentor was set at 28°C, the pH at 6.9, aeration at 0.5 L/min and agitation at 300 rpm. IPTG induction (0.2 mM final concentration) was performed when OD₆₀₀ of the culture reached 7 to 8 and additional LB media and glucose were supplemented. Bacteria were harvested 3 hours after induction, pelleted and frozen at -80°C until use.

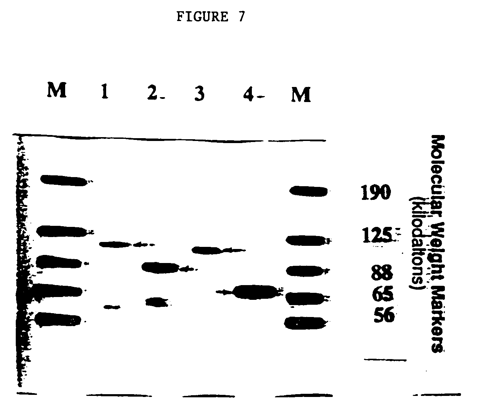

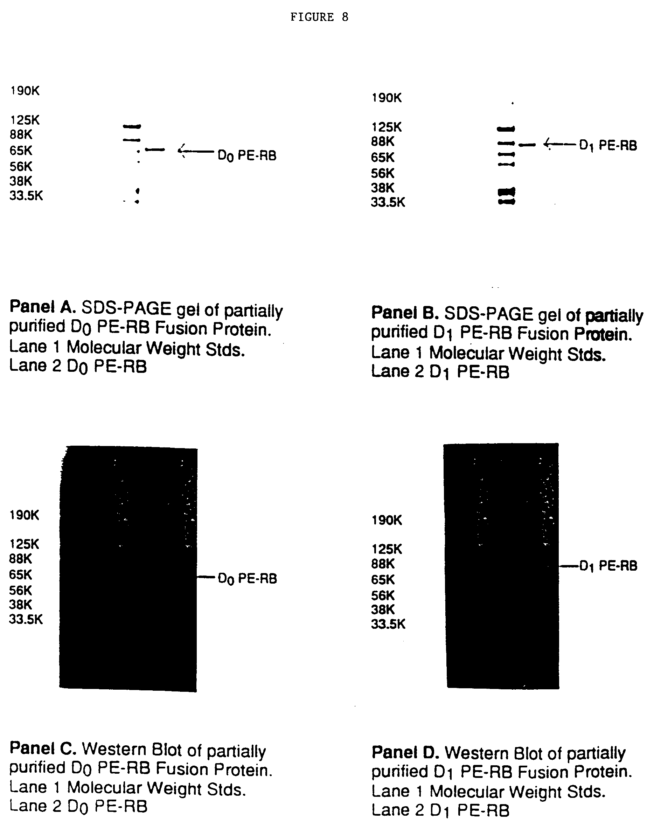

Cell homogenates were prepared using a microfluidizer model M110T at 10,000 psig in the lysis buffer containing 10mM phosphate, 1mM EDTA, and 1mM PMSF at pH 7.5. The resulting microfluidized cell lysate was spun down at 10,000 rpm in a JA-10 rotor for 25 minutes at 4°C. Western blot analysis utilizing ∞-Rb monoclonal antibody 318 of the fusion proteins indicating that they each have the correct molecular weight and react specifically with the Rb moiety at their C-termini is shown in Figure 7.

### EXAMPLE III

### Purification & Biological Activity of the D0 & D1 Fusion Proteins

For D0 and D1, the pellet was resuspended in 2X volumes (in mL) with respect to the wet cell weight (in grams) of lysis buffer and spun down again in the JA-10 rotor for 25 minutes. Hence, 2X volumes refers to this volume of lysis buffer. This washing procedure was repeated three times. The remaining pellet was stirred in 2X volumes of lysis buffer containing 0.1% Tween-80 at 4°C for 1 1/2 - 2 hours and then centrifuged again in the JA-10 rotor.

Following the 0.1% Tween-80 wash step, the pellet was resuspended in 2X volumes of lysis buffer containing 4M urea. This mixture was stirred for at least 1 1/2 - hours at 4°C, followed by centrifugation in the JA-10 rotor as before.

The pellet was resuspended in 2X volumes of lysis buffer containing 8M urea and stirred for at least 1 1/2 - 2 hours at 4°C. The resuspended material was centrifuged again as before, the supernatant contains the partially purified fusion proteins.

Refolding was accomplished by drop-wise addition of the denatured protein solution into a rapidly stirring flask containing 8X - 40X volumes of 10mM phosphate + 150mM NaCl at pH 7.5 (PBS). The resulting protein solution was concentrated and the buffer exchanged in additional PBS to further reduce the urea and PMSF concentrations.

The protein obtained by this method is 80-85% pure as estimated by Coomassie blue staining of a reducing SDS-PAGE gel. Figure 8 (Panels A and B) show SDS-PAGE gel analysis of the purified D0 and D1 proteins. Western blot analysis using ∞Rb Mab 3C8 support the identity of these proteins (Figure 8, Panels C and D).

### EXAMPLE IV

### T-antigen Binding Characteristics of PEA56RBD0

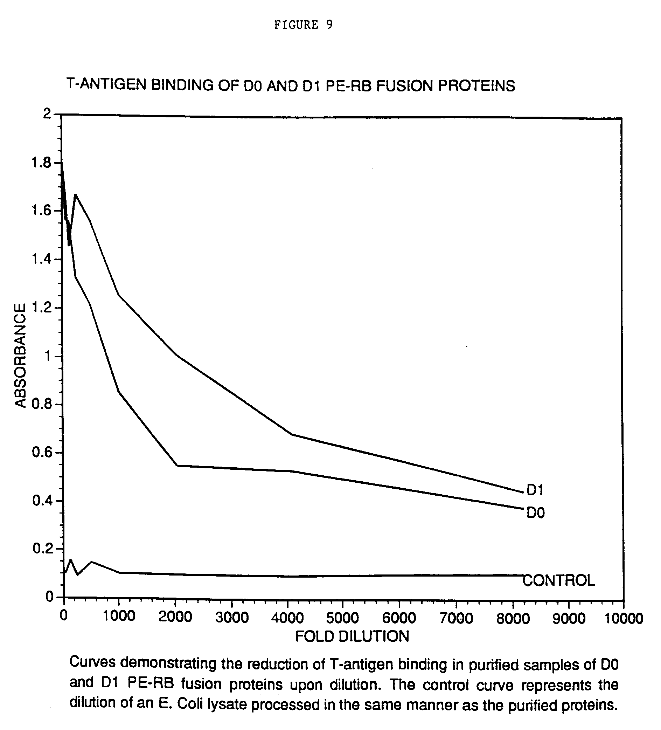

The ability of purified fusion proteins to bind purified viral oncoprotein SV40 T-antigen was determined according to the modified ELISA procedure of Wen et al. Briefly, each well of the 96-well microtiter plate was coated overnight with 0.05 µg of purified Tag. After blocking with BSA (10mg/ml in PBS), purified PEA56RBD0 fusion protein of various concentrations (two-fold dilutions from 2 µg/ml to 0.016 µg/ml) was added to each well. The wells were washed and the Tag-bound RB protein was detected (Figure 9) by the standard chromogenic reaction using biotinylated Mab 3C8, streptavidin-POD 1:2500 dilution, (Boehringer Mannheim GmbH), and ABTS (Sigma, St. Louis, MO) substrate. Color development was measured at 405 nm absorbance by a Coulter microplate autoreader (Molecular Devices).

### EXAMPLE V

### In Vitro Growth Suppression of p110^{RB} Deficient Lung Tumor Cell Lines by Fusion Proteins and Full Length RB (p110^{RB})

Tumor cells used included: the RB negative non-small cell lung carcinoma cell line, (Harbour, J.W. et al., "Abnormalities in structure and expression of the human retinoblastoma gene in SCLC", Science, 241:353-7 (1988)) and the small cell lung carcinoma lines (Hensel, C.H. et al., "Altered structure and expression of the human retinoblastoma susceptibility gene in small-cell lung cancer", Canc. Res., 50:3067-3072 (1990)); and the Rb positive non-small cell lung carcinoma line, (Yokota, J. et al., "Altered expression of the retinoblastoma (RB) gene in a small-cell carcinoma of the lung", Oncogene, 3:471-5 (1988)). These cell lines were from ATCC (Rockville, MD). About 5x10³ cells were seeded into each well in a 96-well plate and were allowed to equilibrate in RPMI media overnight at 37°C. The media was aspirated and 100 µl of fresh RPMI supplemented with 5% fetal calf serum was added to each well. Additionally, fusion protein was added to the media to generate final concentrations ranging from 25 µg/mL to 0.4 µg/mL. Controls which had no analyte addition were also performed. Each incubation was performed in triplicate. The cells were incubated at 37°C in the presence of the analyte for 2 hours. Subsequently, the media was aspirated and replaced with an equal volume of fresh RPMI plus 5% fetal calf serum. The cells were incubated in analyte deficient media for 4 hours. A second 2 hour treatment with the analyte was performed using the same concentration in each well as previous. This was followed by a second 4 hour analyte deficient incubation. The 2 hour treatment was repeated a third time followed again by a 4 hour analyte deficient incubation. Hence, exposure time to the analyte totalled 6 hours.

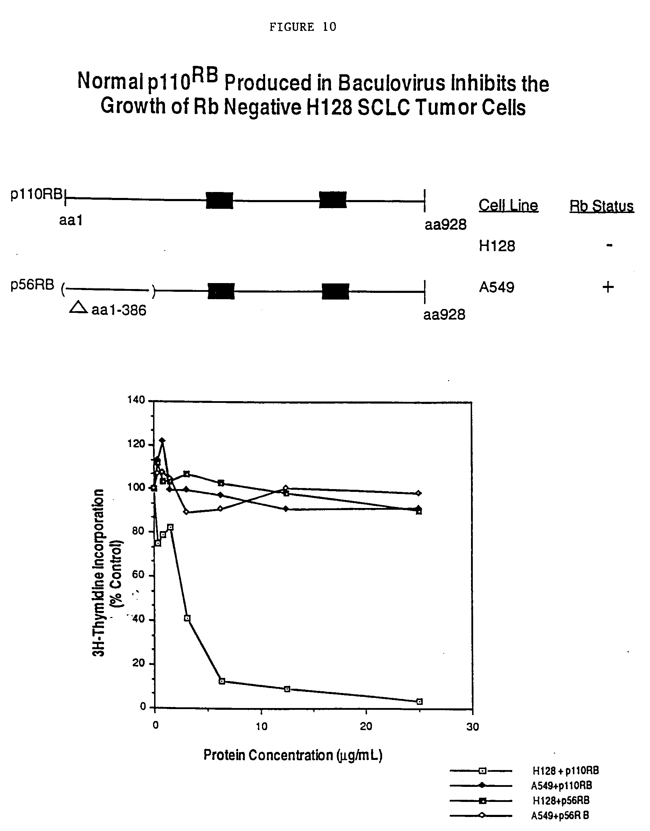

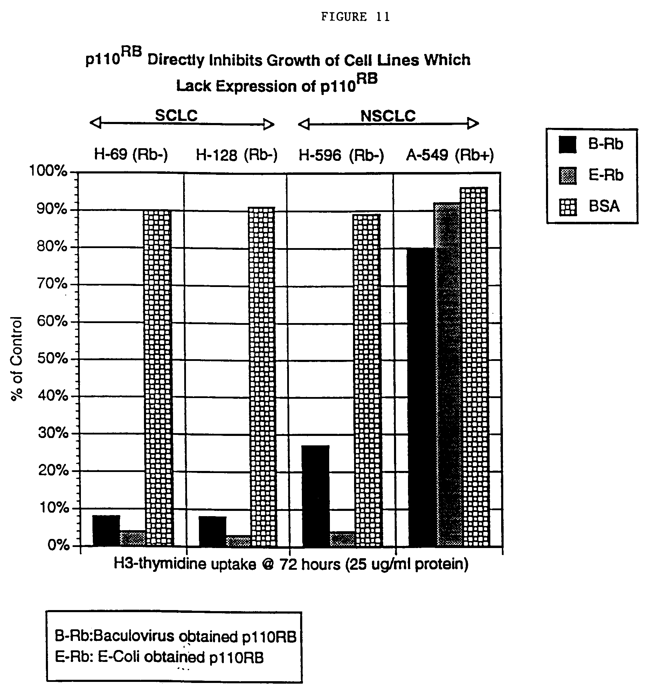

The ³H-thymidine uptake assay was employed for in vitro studies of growth (DNA Synthesis) suppression by RB proteins. For ³H-thymidine uptake studies, the media in each well was removed and replaced by fresh RPMI supplemented with 5% fetal calf serum and 5 µCi/mL ³H-thymidine. Cells were incubated in this environment for 16 hours, after which they were harvested and counted for ³H-thymidine uptake. Inhibition of DNA synthesis by p110^{RB} in p110^{RB}-deficient tumor cell lines is shown in Figures 10 and 11.

## Claims

1. A soluble molecular complex for introducing a cancer suppressor transcription factor into a mammalian cell, the complex comprising a fusion of a ligand for a mammalian cell membrane receptor releasibly bound to the cancer suppressor transcription factor.

2. The soluble molecular complex of claim 1, wherein the cancer suppressor transcription factor is an RB agonist polypeptide.

3. The soluble molecular complex of claim 2, wherein the RB agonist comprises a small molecule derivative of p110^{RB}.

4. The soluble molecular complex of claim 3, wherein the small molecule derivative of p110^{RB} exhibits the biological activity of a soluble full-length retinoblastoma protein.

5. The soluble molecular complex of claim 4, wherein the biological activity includes a nuclear localization signal.

6. The soluble molecular complex of claim 4, wherein the biological activity comprises tumor growth suppression.

7. The soluble molecular complex of claim 3, wherein the small molecule derivative is p56^{RB} and fragments thereof.

8. The soluble molecular complex according to any one of claims 1 to 7 wherein the ligand specifically binds to a cell membrane -COOH receptor or to asialoglycoprotein.

9. The soluble molecular complex of claim 8, wherein the asialoglycoprotein is asialo-GM1 or asialo-GM2.

10. The soluble molecular complex according to any one of claims 1 to 9, wherein the ligand is Pseudomonas exotoxin A or interferon alpha.

11. The soluble molecular complex according to any one of claims 1 to 10 whereby the fusion protein is covalently modified with a hydrophobic monomer.

12. A soluble molecular complex for introducing a cancer suppressor transcription factor to a mammalian cell selected from the group consisting of PEA56RBD0, PEA56RBD1, PEA56RBD2S and FID56RB.

13. A pharmaceutical composition comprising the soluble molecular complex according to any one of claims 1 to 12 and a pharmaceutically acceptable carrier.

14. The pharmaceutical composition of claim 13 for the treatment of retinoblastoma, bladder carcinoma, breast cancer, chronic myelogenous leukemia, acute myelogenous leukemia, testicular tumors, dysplastic and cancerous ulcerative colitis, sporadic sarcomas, prostate carcinoma, osteosarcoma, small-cell lung cancer, synovial sarcoma and other malignancies or hyperproliferative diseases characterized by RB gene dysfunction or alteration of the RB gene product.

15. A method of modifying a cell-regulatory activity of the Rb gene comprising contacting the cell with the soluble molecular complex according to any one of claims 1 to 12, thereby modifying the cell-regulatory activity.

16. The method of claim 15, wherein the ligand releases the RB agonist into the cytoplasm of the mammalian cell.

17. The method of claim 15 or 16, wherein the contacting is effected in vitro.

18. The method according to any one of claims 15 to 17, wherein the regulatory activity comprises tumor growth suppression.
